# EUROPÄISCHE PATENTSCHRIFT

(11) **EP 1 507 515 B1**
(45) Veröffentlichungstag und Bekanntmachung des Hinweises auf die Patenterteilung: **30.03.2011**
(21) Anmeldenummer: 03732496.9
(22) Anmeldetag: 28.05.2003
(51) Int. Cl.: A61K 9/14, A61K 31/385

(54) **FLIESSFÄHIGE, PULVERFÖRMIGE ALPHA-LIPONSÄURE(-DERIVATE) ENTHALTENDE ZUSAMMENSETZUNG**
FREE-FLOWING, POWDERY COMPOSITION CONTAINING ALPHA-LIPONIC ACID (-DERIVATIVES)
COMPOSITION FLUIDE PULVERULENTE CONTENANT DE L'ACIDE ALPHA-LIPOIQUE OU DES DERIVES D'ACIDE ALPHA-LIPOIQUE

(30) Priorität: 29.05.2002 DE 10223882
(43) Veröffentlichungstag der Anmeldung: 23.02.2005
(73) Patentinhaber: AlzChem Trostberg GmbH, 83308 Trostberg (DE)
(72) Erfinder: SCHUHBAUER, Hans, Edmonton ABT6E 5J9 (CA); DREXEL, Claus-Peter, 63263 Neu-Isenburg (DE); FAIROW, Herbert, Clinton, Tolono, IL 61880 (US); KRIMMER, Hans-Peter, 84558 Kirchweidach (DE)
(74) Vertreter: Weiss, Wolfgang
(86) Internationale Anmeldenummer: PCT/EP2003/005663
(87) Internationale Veröffentlichungsnummer: WO 2003/099256

(56) Entgegenhaltungen:
- EP-A- 0 858 802
- DE-A- 19 938 098

## Beschreibung

Gegenstand der vorliegenden Erfindung ist eine fließfähige, pulverfähige α-Liponsäure(-Derivate) enthaltende Zusammensetzung, ein Verfahren zu deren Herstellung und ihre Verwendung.

α-Liponsäure (Thioctsäure, 1,2-Dithiolan-3-pentansäure) ist seit ca. 50 Jahren als Wachstumsfaktor in Mikroorganismen bekannt, sie kommt aber als R-(+)-Enantiomer in geringen Konzentrationen auch in höheren Pflanzen und Tieren vor. α-Liponsäure wirkt physiologisch in hydrophilen und lipophilen Medien als Coenzym der oxidativen Decarboxylierung von α-Ketocarbonsäuren (z.B. Pyruvat, α-Ketoglutarat). Außerdem ist α-Liponsäure auch beim Abbau bestimmter Aminosäuren als Co-Faktor beteiligt. Überdies trägt sie zur Regenerierung von Vitamin C, Vitamin E, Glutathion und CoEnzym Q10 bei. Weiter haben α-Liponsäure und ihr zugehöriger Redoxpartner Dihydroliponsäure stark antioxidative und mitunter auch prooxidative Eigenschaften; oft wird α-Liponsäure daher als "universelles Antioxidans" bezeichnet. Als pharmazeutischer Wirkstoff oder als Nahrungsmittelzusatz kommt racemische α-Liponsäure sowohl als reiner Feststoff im Gemisch mit anderen Komponenten, in festen galenischen Formulierungen, aber auch in Infusionslösungen zum Einsatz. Racemische α-Liponsäure ist zur Behandlung von Lebererkrankungen und Neuropathien (z.B. diabetische Polyneuropathie) zugelassen; ihr Einsatz als effektiver Inhibitor der Replikation von HIV-1-Viren wurde diskutiert (vgl. Klin. Wochenschr. 1991, 69(15), 722-724). Vornehmlich im Anfangsstadium einer entsprechenden klinischen Therapie werden bevorzugt Injektionslösungen von α-Liponsäure eingesetzt. Das R-Enantiomer von α-Liponsäure befindet sich seit Dezember 2000 in Deutschland und seit Mai 2001 in USA in der klinischen Phase II für Anwendungen auf dem Gebiet Typ II Diabetes.

Synthese-Methoden für racemische α-Liponsäure sowie für enantiomerenreine R- oder S-α-Liponsäure sind beispielsweise in Crévisy et al., Eur. J. Org. Chem. 1998, 1949, Fadnavis et al., Tetrahedron Asym. 1998, 9, 4109, Dhar et al., J. Org. Chem. 1992, 57, 1699, Adger et al., J. Chem. Soc. Chem. Commun. 1995, 1563, Dasaradhi et al., J. Chem. Soc. Chem. Commun. 1990, 729, Gopalan et al., J. Chem. Soc. Perkin Trans. I 1990, 1897, Yadav et al., J. Sci. Ind. Res. 1990, 49, 400, Tolstikov et. al., Bioorg. Khim. 1990, 16, 1670, Gopalan et al., Tetrahedron Lett. 1989, 5705, beschrieben oder zusammengefasst.

Verbindungen mit einem niedrigen Festpunkt, wie sie racemische α-Liponsäure mit 60 - 62 °C oder enantiomerenreine R-(+)- oder S-(-)-α-Liponsäure mit 47 - 50 °C darstellen, bilden bei Temperaturen in der Nähe ihres Schmelzpunkts weiche Oberflächen aus, die das Verkleben einzelner Partikel untereinander zur Folge haben. Die Empfindlichkeit von racemischer oder enantiomerenreiner α-Liponsäure gegenüber Licht-und TemperaturEinflüssen sowie eine allgemeine Tendenz zur Polymerisierbarkeit wirken sich zusätzlich negativ aus. Der Grund für diese Empfindlichkeit liegt in der extrem leichten Spaltbarkeit der charakteristischen Disulfidbindung des gespannten fünfgliedrigen Rings in der lipophilen Kette des Moleküls. Mit einer solchen Spaltung geht eine intermolekulare Ausbildung von Disulfidbrücken einher, welche zu dimeren, oligomeren und polymeren Liponsäure-Derivaten führt (vgl. DE-OS 1617740). Dies kann unter Licht- oder Temperatureinfluss geschehen, aber auch durch die Anwesenheit von Nucleophilen (J. Org. Chem. 1969, 34, 3131). Auch die oxidative Zersetzung ist literaturbekannt (J. Org. Chem. 1975, 40, 58-62). Diese Tendenz zur Polymerisierung ist bei den reinen Enantiomeren der α-Liponsäure noch stärker ausgeprägt als beim Racemat. Aufgrund der makroskopischen Eigenschaften dieser Liponsäure-Polymere tendiert das Produkt allgemein zur Bildung von Anbackungen und Verklumpungen. Je höher dabei der Anteil dieser Polymere ist, desto stärker ist die Verklumpungstendenz, und um so ausgeprägter ist die Fließfähigkeit des Produkts herabgesetzt.

Als übliche Reinigungsmethode für rohe α-Liponsäure wird eine Umkristallisation aus organischen Lösemitteln, wie z.B. n-Pentan, Cyclohexan, Methylcyclohexan, Ethylacetat, oder Gemischen von Lösemitteln (z.B. aus Ethylacetat und Hexan), angewendet, wie sie beispielsweise in Brookes et al., J. Chem. Soc. Perkin Trans. 1 1988, 9, Segre et al., J. Am. Chem. Soc. 1957, 3503, Walton et al., J. Am. Chem. Soc. 1955, 77, 5144, Acker et al., J. Am. Chem. Soc. 1954, 76, 6483, beschrieben ist.

Technisch realisiert sind auch Verfahren zur Extraktion und/oder Kristallisation von α-Liponsäure, die den Einsatz organischer Lösemittel mit einer Dielektrizitätskonstante ε von 2.5 bis 5.5 (DE-OS 42 35 912) bzw. 1.95 bis 2.4 (EP-PS 1 100 793) vorsehen.

Ein Aufarbeiten der Mutterlaugen zur Steigerung der Ausbeute und damit der Verfahrens-Effektivität ist technisch sehr aufwändig und führt im Allgemeinen zu einer Verschlechterung der Fließfähigkeit sowie zu einer Verstärkung der Verklumpungstendenz. Als alternative zusätzliche Reinigungsmethode für Liponsäure, die zuvor aus einem Gemisch aus Cyclohexan und Ethylacetat umkristallisiert wurde, wird in DE 197 26 519 A1 eine Behandlung des rohen, mit Liponsäure angereichertem Materials mit flüssigem oder überkritischem Kohlendioxid vorgeschlagen. Gemäß DE-OS 199 38 621 wird durch das Auflösen einer rohen α-Liponsäure in verdünnter, wässriger, alkalischer Lösung, das Abfiltrieren vorhandener fester Verunreinigungen und Wiederansäuern eine kristalline α-Liponsäure erhalten, die sich durch die Abwesenheit von Verunreinigungen wie 1,2,3-Trithian-4-valeriansäure (Epiliponsäure) sowie das Fehlen organischer Lösemittel auszeichnet.

In EP-A 733 363 wird ein Aufbaugranulat der α-Liponsäure beschrieben, das mit Hilfe eines aufwändigen Verfahrens nach Vorlegen von α-Liponsäure in einer Wirbelschicht-Vorrichtung durch Versprühen einer α-Liponsäure-Lösung auf die Vorlage unter gleichzeitiger Entfernung des Lösemittels erhalten wird. Eine derartige α-Liponsäure weist eine spezifische Oberfläche von >0,7 m²/g und einen Anteil an Mesoporen mit einem Durchmesser zwischen 2 und 30 nm auf. Diese hohe spezifische Oberfläche führt in Verbindung mit unterschiedlichen Partikelgrößen häufig zu Entmischungen, wobei sich insbesondere beim Transport, beim Umfüllen oder Fördern die größeren Teilchen schneller bewegen als die kleinen. Auch die Partikelform dieses Aufbaugranulats beeinträchtigt die Fließfähigkeit des Produkts, da eine ungleichmäßige Oberfläche zu einer erhöhten Reibung sowie zu einem vermehrten Verhaken der Teilchen ineinander führt. Überdies bewirkt der Restlösemittelgehalt in derartigen Aufbaugranulaten eine verstärkte Teilchenhaftung im Produkt, die durch Kapillarflüssigkeiten im teilweise oder vollständig gefüllten Mesoporen-Raum vermittelt wird.

Die aus dem Stand der Technik bekannten Verfahren ermöglichen trotz eines erheblichen verfahrenstechnischen Mehraufwandes und bei für eine kommerzielle Produktion von α-Liponsäure ungünstigen Ausbeuten nur eine sehr eingeschränkte und überdies für viele technische Anwendungen unzureichende Verbesserung der Fließfähigkeit des Produkts. Die bislang erhältlichen Qualitäten von α-Liponsäure liegen somit auch nur in einer für die Weiterverarbeitung zu festen Darreichungsformen ungünstigen Form vor. Auch ist die Tatsache hinlänglich bekannt, dass bei der Herstellung von festen Darreichungsformen der α-Liponsäure selbst scheinbar erprobte Rezepturen auch bei Einhaltung aller Verfahrensvorschriften mit α-Liponsäure-Chargen unterschiedlicher Provenienz mitunter unerklärliche Ergebnisse liefern, was sich meist in nicht ausreichenden Produktqualitäten äußert. Überdies ist zwar durchaus anerkannt, dass beispielsweise die Korngrößenverteilung der eingesetzten α-Liponsäure-Charge eine wichtige Kenngröße für die Tablettierbarkeit darstellt; eine entsprechende Spezifizierung der Schlüsselparameter gelang allerdings bislang noch nicht. Wenn nicht alle wichtigen Bindungsmechanismen (Sinterbrücken, Kristallisation und Strukturänderungen, Flüssigkeitsbrücken, chemische Bindungen, Haftung durch Kapillarflüssigkeiten) bekannt sind, können Teilchen-Agglomerationen in der Regel nicht verhindert werden.

Um die durch Partikelhaftung, elektrostatische Haftung, van-der-Waals-Kräfte oder Flüssigkeitsbrücken verursachten Haftkräfte zwischen Partikeln zu vermindern, ist in der Galenik von Wirkstoffen für feste Darreichungsformen der Zusatz unterschiedlichen Arten von Fließhilfsmitteln weit verbreitet. Beispiele solcher Fließhilfsmittel in Form synthetischer Kieselsäuren, die sich zur Verhinderung von Sintervorgängen eignen, sind AEROSIL^{®} 200 oder AEROSIL^{®} 380 (beides nach dem Hochtemperaturhydrolyse-Verfahren hergestellte synthetische, hochdisperse Kieselsäuren der Degussa AG) *(*Pharm. Ind. 1970, 32, 478). Es konnte jedoch nicht vorhergesehen werden, ob der Einsatz von Fließhilfsmitteln in Kombination mit α-Liponsäure zu verbesserten Produktqualitäten führt bzw. welche Spezifikationen geeignete Fließhilfsmittel besitzen müssen.

DE19938098 betrifft ein Verfahren zur Herstellung einer komprimierten Arzneiform mit einem hohen Anteil an Thioctsäure ( alpha - Liponsäure) als Wirkstoff, wobei die Thioctsäure mit Hilfsstoffen verpresst wird.

Für die vorliegende Erfindung hat sich somit die Aufgabe gestellt, eine α-Liponsäure(-Derivate) enthaltende Zusammensetzung bereitzustellen, die möglichst keinerlei Agglomerierungstendenzen zeigt und die auch nicht zu Ver- und Anbackungen neigt. Darüber hinaus sollte sich die Zusammensetzung leicht und ohne großen Aufwand zu festen Darreichungsformen für pharmazeutische, diätetische oder kosmetische Zwecke weiterverarbeiten lassen.

Gelöst wurde diese Aufgabe mit Hilfe einer fließfähigen, pulverförmigen Zusammensetzung, die aus einer racemischen α-Liponsäure, Dihydroliponsäure, enantiomerenreine R-(+)- oder S-(-)-α-Liponsäure oder -Dihydroliponsäure oder deren Mischungen als Liponsäure-Komponente sowie 0,1 bis 25 Gew.-%, bezogen auf das Gesamtgewicht der Zusammensetzung, eines Kieselsäure-basierten Fließhilfsmittels besteht das eine Teilchengröße (x100-Wert) zwischen 50 und 600 µm und eine Stampfdichte von 50 bis 600 g/L aufweist.

Dabei wird unter dem "x100-Wert" im Gegensatz zum d50-Wert verstanden, dass 100 % der eingesetzten Teilchen maximal die jeweilig angegebene Größe aufweisen.

Die beanspruchte Zusammensetzung weist deutlich verbesserte Eigenschaften bezüglich Fließfähigkeit, Fließverhalten, Sintertendenz und Stapelfestigkeit auf und ihr Feststoffhandling beim Umfüllen oder Fördern ist erleichtert. Dies war so nicht zu erwarten.

Insbesondere zeichnet sich die Zusammensetzung durch eine deutlich verbesserte Fließfähigkeit im Vergleich zu Qualitäten des Stands der Technik sowie zusätzlich durch ein signifikant verbessertes Fließverhalten aus. Überraschenderweise wird nämlich in der Zusammensetzung gemäß Erfindung durch Verwendung spezifischer Fließhilfsmittel im Gegensatz zu anderen Fließhilfsmitteln der so genannte "Kernfluss" fast vollständig unterbunden. Damit kommt beim Umfüllen, Schütten oder Entleeren nahezu die gesamte Pulvermenge in Bewegung und die unerwünschte Bildung von "toten Zonen" wird annähernd vollständig unterbunden.

Racemische α-Liponsäure und Dihydroliponsäure, enantiomerenreine R-(+)- oder S-(-)-α-Liponsäure und -Dihydroliponsäure oder deren beliebige Mischungen sind als Liponsäure-Komponenten anzusehen. Des Weiteren kann die α-Liponsäure oder auch deren natürlicher Liponsäure-Redoxpartner Dihydroliponsäure ganz oder teilweise in Form ihrer Salze vorliegen. Als Salze kommen hierbei insbesondere das Natrium-, Kalium-, Ammonium-, Magnesium- oder Kreatinlipoat und/oder das Natrium-, Kalium-, Magnesium- oder Kreatindihydrolipoat in Frage. Darüber hinaus kann die Liponsäure und/oder Dihydroliponsäure ganz oder teilweise als Salz mit basischen Aminosäuren, wie z.B. Lysin, Arginin oder Ornithin, vorliegen.

Wie beschrieben, konnte die dieser Erfindung zugrunde liegende Aufgabe durch den Zusatz bestimmter Fließhilfsmittel zu α-Liponsäure gelöst werden. Dabei zeigten sich als Fließhilfsmittel hydrophile sprühgetrocknete Fällungskieselsäuren, wie z.B. SIPERNAT^{®} 22 S,SIPERNAT^{®} 50 S und SIPERNAT^{®} 500 LS (Degussa AG), oder hydrophile hochdisperse Kieselsäuren, wie z.B. vom Typ AEROSIL^{®} 200, sowie hydrophobe Kieselsäuren, wie z.B. vom Typ AEROSIL^{®} R972, als besonders geeignet, die angestrebten Qualitäts- und Handlingsverbesserungen bei α-Liponsäure zu erzielen. Die hydrophilen Fällungskieselsäuren des Typs SIPERNA^{®} 22 S, SIPERNAT^{®} 50 S und SIPERNAT^{®} 500 LS entsprechen im Übrigen auch den Anforderungen für den Lebensmittelzusatzstoff E 551 der EU-Richtlinie 2000/63/EU und sind somit beispielsweise für den Einsatz in Trockenlebensmitteln in Pulverform (einschließlich Zuckerarten) bis zu einer Höchstmenge von 10 g/kg sowie für pulverförmige Aromen bis zu einer Höchstmenge von 1000 mg/kg zugelassen. Als ebenfalls geeignete Fließhilfsmittel im Sinne der vorliegenden Erfindung haben sich amorphe Kieselgele gezeigt, wie sie beispielsweise solche des Typs SYLOX 2, 15 & T450 (GRACE Davison, Columbia) darstellen.

Der Anteil der Fließhilfsmittel-Komponente in der erfindungsgemäßen Zusammensetzung sollte zwischen 0,1 und 25 Gew.-%, bezogen auf das Gesamtgewicht des erfindungsgemäßen Produkts, betragen. Als bevorzugte Gewichtsanteile des Fließhilfsmittels sind Bereiche zwischen 1,0 und 10,0 Gew.-% und besonders bevorzugt solche zwischen 1,5 und 5,0 Gew.-%, anzusehen. Am meisten bevorzugt sind Bereiche von 2,0-3,0 Gew.-%. Alle hier angegebenen Gewichtsanteile bezüglich des Fließhilfsmittels sind auf racemische oder optisch reine α-Liponsäure bezogen. Dies bedeutet, dass bei der Verwendung von Liponsäure-Derivaten oder Salzen die angegebenen Dosiermengen denen der freien Liponsäure entsprechen, weshalb sie dem veränderten Molekulargewicht angepasst werden müssen.

Der Anteil der Wirkstoff-Komponente in der erfindungsgemäßen Zusammensetzung beträgt vorzugsweise 50-99,9 Gew.-%. Besonders bevorzugt ist ein Anteil der Wirkstoff-Komponente von 75-99,0 Gew.%, am meisten bevorzugt ist ein Anteil von 90,0-98,0 Gew.-%.

Die hydrophilen oder hydrophoben Fließhilfsmittel weisen üblicherweise eine ausgezeichnete Eigenfließfähigkeit auf und können durch Vermahlen hinsichtlich ihrer erfindungswesentlichen Merkmale Teilchengröße und Stampfdichte gezielt eingestellt werden.

In diesem Zusammenhang haben sich bezüglich des Fließhilfsmittels Teilchengrößen (x100-Wert) als besonders geeignet gezeigt, die zwischen 50 und 600 µm liegen. Als bevorzugte Bereiche hinsichtlich des Parameters Stampfdichte haben sich 75 bis 100 g/L gezeigt, wobei anzumerken ist, dass sich diese Angaben auf nicht gesiebte Fließhilfsmittel beziehen.

Für bestimmte Anwendungsfälle kann auch die spezifische Oberfläche des Fließhilfsmittels von Bedeutung sein, weshalb die vorliegende Erfindung Bereiche zwischen 30 und 1.000 m²/g und insbesondere solche zwischen 190 und 450 m²/g als bevorzugt ansieht. Die spezifischen Oberflächen wurden dabei mit einem Areameter unter Anwendung von Stickstoff ermittelt.

Ebenfalls von Bedeutung kann der pH-Wert sein, den die jeweils eingesetzten Fließhilfsmittel in wässriger Lösung erzielen. Hierbei sieht die vorliegende Erfindung vor, dass Fließhilfsmittel bevorzugt werden, die in 5 % iger wässriger Lösung einen pH-Wert von 3,0 bis 11,5 und insbesondere von 6,0 bis 7,0 ergeben.

Ein weiterer Vorteil der erfindungsgemäßen Zusammensetzung ist in der Tatsache zu sehen, dass die eingesetzte Liponsäure-Komponente beliebigen Ursprungs sein kann, d.h. die verbesserte Fließfähigkeit stellt sich unabhängig von der zur Herstellung verwendeten Syntheseroute ein. Auch der Lösemittel- und Polymergehalt der Liponsäure-Komponente ist im Rahmen der üblichen Spezifikationen ohne Belang. Für die Zusammensetzung gemäß vorliegender Erfindung eignen sich auch solvensfreie α-Liponsäuren, wie sie in DE-OS 199 38 621 beschrieben sind, oder nach DE 197 26 519 A1, DE - OS 42 35 912, DE 198 34 608 A1, EP 586 987, DE 198 45 517 A1, EP 733 363 oder EP 593 896 hergestellte oder gereinigte racemische oder enantiomerenreine α-Liponsäuren.

Neben der beschriebenen Zusammensetzung beansprucht die vorliegende Erfindung auch ein Verfahren zu deren Herstellung, bei dem
(a) die pulverförmige Liponsäure-Komponente mit dem festen Fließhilfsmittel gemischt wird und anschließend
(b) aus dem erhaltenen Feststoffgemisch Partikel >800 µm abgetrennt werden.

Im Rahmen der vorliegenden Erfindung können dabei die Verfahrensschritte (a) und/oder (b) beliebig oft wiederholt werden.

Auch die Unkompliziertheit und Direktheit des vorliegenden Verfahrens stellen erhebliche Vorzüge der vorliegenden Erfindung dar. Kleinere Mengen der erfindungsgemäßen Zusammensetzung können auf einfache Weise durch 3-minütiges Mischen in einem Turbula-Mischer und anschließendes Absieben hergestellt werden. Für technische Ansätze sind im Verfahrensschritt (a) Mischwerkzeuge mit geringen Scherkräften vorzuziehen, wie sie Freifallmischer in Form von Trommel-, V- und Taumel-Mischern, horizontal arbeitende Paddel- und Pflugscharmischer (z.B. Lödige-Mischer), vertikal arbeitende Konusmischer, insbesondere mit flach geschnittenen Schnecken des Nauta-Typs, oder Eirich-Mischer mit diagonalen Mischwerkzeugen darstellen; geeignet sind auch Mischer mit schnell rotierenden Mischwerkzeugen, Mischer mit pulsierender Luft sowie Schnecken- oder Doppelschneckenmischer. Kurze Mischzeiten von 5 bis 30 min Dauer und besonders 10 bis 15 Minuten sowie eine möglichst feine Verteilung der α-Liponsäure können insgesamt von Vorteil sein. Empfehlenswert kann deshalb auch der Eintrag der Liponsäure-Komponente durch Versprühen einer Lösung der Liponsäure-Komponente auf einer Trägerkieselsäure unter gleichzeitiger Entfernung des Lösemittels sein. Alternativ ist auch das Zudosieren des Fließhilfsmittels im Sprühturm eine besonders bevorzugte Ausführungsform der vorliegenden Erfindung.

Für das vorliegende Verfahren spielt es nämlich im Übrigen keine Rolle, in welcher Reihenfolge das Fließhilfsmittel und die Liponsäure-Komponente zugegeben werden. Die vorliegende Erfindung sieht allerdings in Verfahrensstufe (a) bevorzugt den portionsweisen Zusatz mindestens einer der beiden Komponenten vor. Die Zugabe der Komponenten kann aber insgesamt beliebig häufig, anteilig und in beliebiger Schnelligkeit erfolgen.

Mit Hilfe eines einfachen Verfahrens kann somit die Liponsäure-Komponente mit der hydrophilen oder hydrophoben Fließhilfsmittel-Komponente unter Einwirkung geringer Scherkräfte schnell und effektiv vermischt werden.

Eine u.a. derartig hergestellte Liponsäure-haltige Zusammensetzung lässt sich leicht zu entsprechenden Darreichungsformen weiterverarbeiten, wofür die vorliegende Erfindung die Verwendung in festen Darreichungsformen für pharmazeutische, diätetische und/oder kosmetische Anwendungen vorsieht. Insbesondere lassen sich gepresste Applikationsformen und besonders bevorzugt Tabletten mit hohem Wirkstoffgehalt für perorale Anwendungen, wie sie beispielsweise im Rahmen einer klinischen Therapie bei diabetischer Polyneuropathie oder Typ 2 Diabetes eingesetzt werden, in vereinfachter Weise herstellen.

Ergänzend berücksichtigt die Erfindung auch die Verwendung der Zusammensetzung zur Herstellung von Infusionslösungen.

Im Vergleich zu den bislang bekannten Liponsäure-haltigen Zusammensetzungen des Stands der Technik weist die erfindungsgemäße Zusammensetzung generell verbesserte Eigenschaften bezüglich Fließfähigkeit, Fließverhalten, Sintertendenz und Stapelfestigkeit auf. Darüber hinaus ist das Feststoffhandling bei Umfüllen, Schütten und/oder Fördern signifikant erleichtert, weshalb die vorliegende Erfindung eine erhebliche Verbesserung der bislang bekannten Liponsäure-haltigen Zusammensetzungen darstellt.

Diese Vorteile der fließfähigen, pulverförmigen α-Liponsäure(-Derivate) enthaltenden Zusammensetzung sollen die nachfolgenden Beispiele verdeutlichen.

### Beispiele

Zur Beurteilung der Produkteigenschaften wurden folgende Methoden eingesetzt:

### (a) Fließfähigkeit durch Auslaufgeräte

Zur Bestimmung der Fließfähigkeit ohne Druckbehandlung wurden silikonisierte Glasauslaufgefäße mit unterschiedlichen Auslaufdurchmessern verwendet *(Seifen, Öle, Fette, Wachse 1968, 94, 12)*. Die Bewertung erfolgte entsprechend den (Schul-)Noten: 1 = sehr gutes Fließverhalten (das zu untersuchende Pulver fließt ohne Stocken aus dem Auslaufgerät Nr. 1 mit der kleinsten Auslauföffnung) bis zur Note 6 = ungenügendes Fließverhalten (das Pulver fließt auch durch das Messgefäß Nr. 5 mit der größten Auslauföffnung nicht mehr aus). Das Messverfahren wurde bei stets gleicher Reihenfolge der Auslaufgefäße 1 bis 6 durchgeführt. Bestimmt wurde das Messgefäß, bei dem die pulverförmige Zusammensetzung gerade noch ohne Stocken ausfließt (Tabelle 1).

**Tabelle 1 Bewertung der Fließfähigkeit mit Glasauslaufgefäßen**

| Gefäß-Nr. | Auslaufweite ø [mm] | Bewertung bei noch stockfreiem Durchlauf des Pulvers |
|---|---|---|
| 1 | 2.5 | sehr gut |
| 2 | 5 | gut |
| 3 | 8 | im ganzen gut |
| 4 | 12 | eben ausreichend |
| 5 | 18 | mangelhaft |
| 6 | | ungenügend (Pulver läuft nicht durch Nr. 5) |

| | | |
|---|---|---|
| (h = 80 mm, ø (innen) = 38 mm) | | |

### (b) Fließfähigkeit durch Schüttkegelhöhe

Über einem Metallvollzylinder mit einem Durchmesser von 50 mm und ca. 80 mm Höhe wurde in einem Abstand von 30-100 mm ein Metallsieb befestigt. Der Abstand zwischen Metallsieb und Metallzylinder richtete sich nach der Fließfähigkeit des zu messenden Pulvers und war etwas größer als die Schüttkegelhöhe des am schlechtesten fließenden Pulvers der jeweiligen Prüfreihe. In dieser Höhe wurde das Sieb fixiert, das Pulver darauf geschüttet und manuell mit Hilfe eines Spachtels langsam durch das Sieb passiert. Das herabfallende Pulver baut so auf dem Metallzylinder einen Schüttkegel auf. Das Pulver wurde dann solange durch das Sieb passiert, bis sich auf dem Zylinder ein geometrisch gleichmäßig geformter Kegel ausgebildet hat. Nun wurde das Sieb entfernt und die Höhe des Schüttkegels gemessen. Aus der Höhe des Schüttkegels und dem Durchmesser des Metallzylinders kann der Böschungswinkel des geprüften Pulvers bestimmt werden. Da der Durchmesser des Kegels konstant ist, kann man auch die Höhe des Schüttkegels als direktes Maß für die Fließfähigkeit heranziehen. Sehr gut fließfähige Pulver haben eine Schüttkegelhöhe von 15-20 mm; Pulver mit einer Schüttkegelhöhe von >50 mm haben ein schlechtes Rieselverhalten (Tabelle 2).

**Tabelle 2 Bewertung der Fließfähigkeit mit Schüttkegelhöhe**

| Schüttkegelhöhe [mm] | Beurteilung | Note |
|---|---|---|
| <20 | sehr gut | 1 |
| 21-30 | gut | 2 |
| 31-40 | eben ausreichend | 3 |
| 41-50 | mangelhaft | 4 |
| >50 | ungenügend | 5 |

### (c) Druckfestigkeit

Pulverförmige Produkte neigen bei Stapelung in Säcken, Trommeln oder Silos zum Zusammenbacken. Zur Beurteilung ihrer "Stapelfestigkeit" dient der folgende Test: In einem Stahl-Zylinder von 50 mm Innendurchmesser wird das zu prüfende Pulver etwa 20 mm hoch eingefüllt und mit einem Druckstempel von 1,2 kg Gewicht sowie einem definierten Auflagegewicht belastet. Die Dauer der Druckbelastung kann der jeweils vorliegenden Beanspruchung des Pulvers hinsichtlich Verpackung, Transport und Lagerung entsprechend gewählt werden. Das bei dieser Prüfanordnung verwendete Auflagegewicht betrug etwa 0,16-0,17 kg/cm², was dem Gewicht von 10 bis 12 aufeinander liegenden Säcken gegebener Größe mit einem Füllgewicht von je 50 kg entspricht. Nach einer Druckbelastung von 24 Stunden Dauer wurde das Auflagegewicht entfernt, die beiden Zylinder wurden von Hand vorsichtig um 180° gedreht und die Pulvertablette mit Hilfe des Druckstempels aus der Hülse herausgedrückt. Die Härte der Pulvertablette wird als Maß für die Druckfestigkeit angesehen; sie kann subjektiv oder mit Hilfe eines Walzensiebs gemessen werden: Zur Messung wurde die Pulvertablette in ein zylinderförmiges Walzensieb gelegt. Mit Hilfe eines Motors wurde das Walzensieb mit 60 U/min rotiert und die Zeit bestimmt, nach der die Tablette nur noch das halbe Gewicht besaß. Dazu befand sich unterhalb des Siebs eine Waage, die das abgeriebene Pulvergewicht anzeigte.

**Tabelle 3 Visuelle Bewertung der Druckfestigkeit**

| Wertungsnote | Verhaltenskennzeichen |
|---|---|
| 1 = sehr gut | völlig unverändert und durch Auslaufgefäß Nr. 2 (Tabelle 1) glatt fließend |
| 2 = gut | teilweise lose haftend, leicht in Originalzustand zerfallend |
| 3 = im ganzen gut | locker geformt; bei leichtem Fingerdruck weitestgehend pulvrig zerfallend |
| 4 = ausreichend | locker verbacken; mit Fingerprobe noch feinst zerfallend |
| 5 = mangelhaft | halbfest verbackend; mit Fingerprobe nicht mehr feinst zerfallend |
| 6 = ungenügend | fest geformt |

### Versuchsergebnisse

Die Vergleichsbeispiele 1 bis 2 wurden mit Zusammensetzungen durchgeführt, die ausschließlich nach dem Stand der Technik hergestellte Liponsäure enthalten. (Beispiel 1: gemäß EP 593 896; Beispiel 2: gemäß DE-OS 199 38 621)

Die Vergleichsbeispiele 3 bis 5 wurden mit Zusammensetzungen durchgeführt, die nach dem Stand der Technik gemäß Beispiel 2 hergestellte Liponsäure und nicht erfindungsgemäße Fließhilfsmittel enthielten.

Die Erfindungsbeispiele 6 bis 19 wurden mit erfindungsgemäßen Zusammensetzungen durchgeführt, die gemäß Beispiel 2 hergestellte Liponsäure und erfindungsgemäße Fließhilfsmittel enthielten.

Alle mit Fließhilfsmittel kombinierten Liponsäure-Zusammensetzungen wurden durch 3-minütiges Mischen in einem Turbula-Mischer, gefolgt von einem Absieben über ein 600 µm-Sieb hergestellt.

Bezüglich der Fließnote werden in den Beispielen 6-19 durchgängig wesentlich bessere Ergebnisse erzielt als in Beispiel 2 (Kontrolle).

Als Kontrolle für die Schüttkegelhöhe dient Vergleichsbeispiel 5. In den Beispielen 6-17 werden diesbezüglich ebenfalls durchgängig bessere Ergebnisse erzielt.

Die Druckfestigkeit nimmt in den Erfindungsbeispielen konzentrationsabhängig zu. Bei Liponsäure-Anteilen von 3,0 Gew.-% beträgt die Zunahme mindestens 1,5 Noten bezüglich Beispiel 2 (Beispiel 19: bei 2,0 Gew.-% 2,5 Noten).

**Tabelle 4 Ergebnisse**

| # | Fließhilfsmittel | Gewichts-Anteil | Fließnote | Schüttkegelhöhe [mm] | Druckfestigkeit |
|---|---|---|---|---|---|
| 1 | n/a | n/a | 4 | 30 | 4-5 |
| 2 | n/a | n/a | 6 | nicht messbar, Kegel bricht ab | 6 |
| 3 | Sipernat^{®} 320 | 1.0 | 5 | nicht messbar, Kegel bricht ab | 5-6 |
| 4 | | 1.5 | 5 | | 5-6 |
| 5 | | 2.0 | 4 | 29 | 5 |
| 6 | Sipernat^{®} 22S | 1.0 | 2 | 25 | 6 |
| 7 | | 1.5 | 2 | 22 | 5-6 |
| 8 | | 2.0 | 2 | 21 | 5 |
| 9 | | 3.0 | 2 | 20 | 4-5 |
| 10 | Sipernat^{®} 50S | 1.0 | 2 | 23 | 5 |
| 11 | | 1.5 | 2 | 21 | 4-5 |
| 12 | | 2.0 | 2 | 19 | 4 |
| 13 | | 3.0 | 2 | 19 | 4 |
| 14 | Sipernat^{®} 500LS | 1.0 | 2 | 22 | 4-5 |
| 15 | | 1.5 | 2 | 19 | 4 |
| 16 | | 2.0 | 2 | 19 | 3-4 |
| 17 | | 3.0 | 2 | 20 | 3- |
| 18 | Silox^{™} 2 | 1.5 | 4 | n/a | 4-5 |
| 19 | | 2.0 | 3 | n/a | 3-4 |

| | | | | | |
|---|---|---|---|---|---|
| "n/a": nicht ermittelbar | | | | | |

## Patentansprüche

1. Fließfähige, pulverförmige Zuammensetzung,
**dadurch gekennzeichnet,**
**dass** sie aus einer racemischen α-Liponsäure, Dihydroliponsäure, enantiomerenreine R-(+)- oder S-(-)-α-Liponsäure oder -Dihydroliponsäure oder deren Mischungen als Liponsäure-Komponente sowie 0,1 bis 25 Gew.-%, bezogen auf das Gesamtgewicht der Zusammensetzung, eines Kieselsäure-basierten Fließhilfsmittels besteht, das eine Teilchengröße (x100-Wert) zwischen 50 und 600 µm und eine Stampfdichte von 50 bis 600 g/l aufweist.

2. Zusammensetzung nach Anspruch 1,
**dadurch gekennzeichnet,**
**dass** die Liponsäure-Komponente zumindest teilweise in Form von Salzen vorliegt, insbesondere als Natrium-, Kalium-, Ammonium-, Magnesium- oder Kreatin-Lipoat und/oder als Natrium-, Kalium-, Magnesium- oder Kreatin-Dihydrolipoat, und/oder ganz oder teilweise als Salz mit basischen Aminosäuren, wie z.B. Lysin, Arginin oder Omithin.

3. Zusammensetzung nach einem der Ansprüche 1 bis 2,
**dadurch gekennzeichnet,**
**dass** sie das Fließhilfsmittel in Anteilen von 1,0 bis 10,0 Gew.-% und besonders bevorzugt in Anteilen von 1,5 bis 5,0 Gew.-%, jeweils bezogen auf das Gesamtgewicht der Zusammensetzung, enthält.

4. Zusammensetzung nach einem der Ansprüche 1 bis 3,
**dadurch gekennzeichnet,**
**dass** es sich beim Fließhilfsmittel um eine hydrophile sprühgetrocknete Fällungskieselsäure, um eine hydrophile hochdisperse Kieselsäure oder um eine hydrophobe Kieselsäure handelt.

5. Zusammensetzung nach einem der Ansprüche 1 bis 4,
**dadurch gekennzeichnet,**
**dass** das Fließhilfsmittel ein amorphes Kieselgel darstellt.

6. Zusammensetzung nach einem der Ansprüche 1 bis 5,
**dadurch gekennzeichnet,**
**dass** das Fließhilfsmittel eine Teilchengröße (x100-Wert) zwischen 50 und 400 µm, aufweist.

7. Zusammensetzung nach einem der Ansprüche 1 bis 6,
**dadurch gekennzeichnet,**
**dass** das Fließhilfsmittel eine Stampfdichte von 75 bis 100 g/L besitzt.

8. Zusammensetzung nach einem der Ansprüche 1 bis 7,
**dadurch gekennzeichnet,**
**dass** das Fließhilfsmittel eine spezifische Oberfläche von 30 bis 1000 m²/g, insbesondere von 190 bis 450 m²/g, besitzt.

9. Zusammensetzung nach einem der Ansprüche 1 bis 8,
**dadurch gekennzeichnet,**
**dass** das Fließhilfsmittel in 5 %iger wässriger Lösung einen pH-Wert von 3,0 bis 11,5, insbesondere von 6,0 bis 7,0, bewirkt.

10. Verfahren zur Herstellung einer Zusammensetzung nach einem der Ansprüche 1 bis 9,
**dadurch gekennzeichnet,**
**dass**
(a) die pulverförmige Liponsäure-Komponente mit dem festen Fließhilfsmittel gemischt wird und
(b) aus dem erhaltenen Feststoffgemisch Partikel >800 µm abgetrennt werden, wobei die Verfahrensschritte (a) und/oder (b) beliebig oft wiederholt werden können.

11. Verfahren nach Anspruch 10,
**dadurch gekennzeichnet,**
**dass** für die Verfahrensstufe (a) Freifallmischer, wie z.B. Trommel-, V- und Taumel-Mischer, horizontal arbeitende Paddel- oder Pflugscharmischer, wie z.B. Lödige-Mischer, vertikal arbeitende Konusmischer, insbesondere mit flach geschnittenen Schnecken des Nauta-Typs, oder Eirich-Mischer mit diagonalen Mischwerkzeugen eingesetzt werden.

12. Verfahren nach einem der Ansprüche 10 oder 11,
**dadurch gekennzeichnet,**
**dass** in Verfahrensstufe (a) mindestens eine der beiden Komponenten portionsweise dem Mischprozess zugesetzt wird.

13. Verwendung der Zusammensetzung nach einem der Ansprüche 1 bis 9 in festen Darreichungsformen zur Herstellung eines Mittels für pharmazeutische, diätetische und/oder kosmetische Anwendungen.

14. Verwendung nach Anspruch 13, zur Herstellung von gepressten Applikationsformen, insbesondere von Tabletten für perorale Verwendungszwecke.

15. Verwendung nach Anspruch 13, zur Herstellung von Infusionslösungen.

## Claims

1. Free-flowing, powdery composition,
**characterized in that**
it consists of a racemic α-lipoic acid, dihydrolipoic acid, enantiopure R-(+)- or S-(-)-α-lipoic acid or -dihydrolipoic acid or mixtures thereof as lipoic acid component and from 0.1 to 25% by weight, based on the total weight of the composition, of a silica-based flow aid which has a particle size (x100 value) of between 50 and 600 µm and a tamped density of from 50 to 600 g/l.

2. Composition according to Claim 1,
**characterized in that**
the lipoic acid component is at least partly in the form of salts, in particular as sodium, potassium, ammonium, magnesium or creatine lipoate and/or as sodium, potassium, magnesium or creatine dihydrolipoate, and/or wholly or partly as salt with basic amino acids, such as, for example, lysine, arginine or ornithine.

3. Composition according to either of Claims 1 to 2,
**characterized in that**
it comprises the flow aid in proportions of from 1.0 to 10.0% by weight and particularly preferably in proportions of from 1.5 to 5.0% by weight, in each case based on the total weight of the composition.

4. Composition according to any of Claims 1 to 3,
**characterized in that**
the flow aid is a hydrophilic spray-dried precipitated silica, a hydrophilic colloidal silica or a hydrophobic silica.

5. Composition according to any of Claims 1 to 4,
**characterized in that**
the flow aid represents an amorphous silica gel.

6. Composition according to any of Claims 1 to 5,
**characterized in that**
the flow aid has a particle size (x100 value) of between 50 and 400 µm.

7. Composition according to any of Claims 1 to 6,
**characterized in that**
the flow aid has a tamped density of from 75 to 100 g/l.

8. Composition according to any of Claims 1 to 7,
**characterized in that**
the flow aid has a specific surface area of from 30 to 1000 m²/g, in particular from 190 to 450 m²/g .

9. Composition according to any of Claims 1 to 8,
**characterized in that**
the flow aid brings about a pH of from 3.0 to 11.5, in particular of from 6.0 to 7.0, in a concentration of 5% in aqueous solution.

10. Process for producing a composition according to any of Claims 1 to 9,
**characterized in that**
(a) the powdered lipoic acid component is mixed with the solid flow aid, and
(b) particles of > 800 µm are removed from the resulting solid mixture,
where process steps (a) and/or (b) can be repeated as often as desired.

11. Process according to Claim 10,
**characterized in that**
free-fall mixers such as, for example, drum, V and tumbler mixers, horizontally operating paddle or plowshare mixers such as, for example, Lödige mixers, vertically operating conical mixers, in particular with flat-cut screws of the Nauta type, or Eirich mixers with diagonal mixing implements are employed for process stage (a).

12. Process according to either of Claims 10 or 11,
**characterized in that**
at least one of the two components is added in portions to the mixing procedure in process stage (a).

13. Use of the composition according to any of Claims 1 to 9 in solid dosage forms for producing a composition for pharmaceutical, dietetic and/or cosmetic applications.

14. Use according to Claim 13, for producing compressed administration forms, in particular tablets for oral use purposes.

15. Use according to Claim 13 for producing solutions for infusion.

## Revendications

1. Composition pulvérulente fluide **caractérisée en ce qu'**elle consiste en un acide α-lipoïque racémique, un acide dihydrolipoïque racémique, un acide R-(+)- ou S-(-)-α-lipoïque énantiomériquement pur ou un acide R-(+)- ou S-(-)-dihydrolipoïque énantiomériquement pur ou leurs mélanges comme composant acide lipoïque ainsi qu'en 0,1 à 25 % en poids par rapport au poids total de la composition, d'un adjuvant d'écoulement à base d'acide silicique, qui présente une taille de particule (valeur x 100) entre 50 et 600 µm et une masse volumique tassée de 50 à 600 g/l.

2. Composition selon la revendication 1 **caractérisée en ce que** le composant acide lipoïque est au moins en partie sous forme de sels, en particulier sous forme de lipoate de sodium, potassium, ammonium, magnésium ou créatine et/ou sous forme de dihydrolipoate de sodium, potassium, magnésium ou créatine, et/ou en totalité ou en partie sous forme de sel avec des aminoacides basiques, comme la lysine, l'arginine ou l' ornithine, par exemple.

3. Composition selon l'une des revendications 1 à 2 **caractérisée en ce qu'**elle contient l'adjuvant d'écoulement en proportions de 1,0 à 10,0 % en poids et de manière particulièrement préférée en proportions de 1,5 à 5,0 % en poids, dans chaque cas par rapport au poids total de la composition.

4. Composition selon l'une des revendications 1 à 3 **caractérisée en ce que** l'adjuvant d'écoulement est un acide silicique précipité séché par pulvérisation hydrophile, un acide silicique hautement dispersé hydrophile ou un acide silicique hydrophobe.

5. Composition selon l'une des revendications 1 à 4 **caractérisée en ce que** l'adjuvant d'écoulement est un gel de silice amorphe.

6. Composition selon l'une des revendications 1 à 5 **caractérisée en ce que** l'adjuvant d'écoulement présente une taille de particule (valeur x 100) entre 50 et 400 µm.

7. Composition selon l'une des revendications 1 à 6 **caractérisée en ce que** l'adjuvant d'écoulement possède une masse volumique tassée de 75 à 100 g/L.

8. Composition selon l'une des revendications 1 à 7 **caractérisée en ce que** l'adjuvant d'écoulement possède une surface spécifique de 30 à 1000 m²/g, en particulier de 190 à 450 m²/g.

9. Composition selon l'une des revendications 1 à 8 **caractérisée en ce que** l'adjuvant d'écoulement produit en solution aqueuse à 5 % un pH de 3,0 à 11,5, en particulier de 6,0 à 7,0.

10. Procédé pour produire une composition selon l'une des revendications 1 à 9 **caractérisé en ce que**
(a) le composant acide lipoïque pulvérulent est mélangé avec l'adjuvant d'écoulement solide et
(b) les particules > 800 µm sont séparées du mélange de solides obtenu,
où les étapes de procédé (a) et/ou (b) peuvent être répétées aussi souvent qu'on le souhaite.

11. Procédé selon la revendication 10 **caractérisé en ce que** des mélangeurs à chute libre, comme par exemple des mélangeurs à tambour, en V et oscillants, des mélangeurs à ailettes ou à soc fonctionnant horizontalement, comme par exemple des mélangeurs Lödige, des mélangeurs à cône fonctionnant verticalement, en particulier avec des vis taillées plates du type Nauta, ou des mélangeurs Eirich à outils mélangeurs diagonaux sont utilisés pour l'étape de procédé (a).

12. Procédé selon l'une des revendications 10 ou 11 **caractérisé en ce qu'**au moins l'un des deux composants est ajouté par portions au processus de mélange dans l'étape de procédé (a).

13. Utilisation de la composition selon l'une des revendications 1 à 9 dans des formes d'administration solides pour la production d'un agent pour des applications pharmaceutiques, diététiques et/ou cosmétiques.

14. Utilisation selon la revendication 13 pour la production de formes d'application compressées, en particulier de comprimés à des fins d'utilisation perorale.

15. Utilisation selon la revendication 13 pour la production de solutions pour perfusion.
